Europäisches Patentamt

European Patent Office

Office européen des brevets

(11) Publication number: **0 464 549 A1**

# EUROPEAN PATENT APPLICATION

(21) Application number: **91110361.2**

(22) Date of filing: **24.06.91**

(51) Int. Cl.⁵: **C07K 7/10, A61K 37/02**

(30) Priority: **26.06.90 JP 165738/90**

(43) Date of publication of application:
**08.01.92 Bulletin 92/02**

(84) Designated Contracting States:
**AT BE CH DE DK ES FR GB GR IT LI LU NL SE**

(71) Applicant: **SANWA KAGAKU KENKYUSHO CO., LTD.**
**No. 35, Higashi-sotobori-cho, Higashi-ku Nagoya, Aichi-ken(JP)**

(72) Inventor: **Kurono, Masayasu, c/o Sanwa Kagaku Kenkyusho Co.**
**Limited, 35, Higashi-sotobori-cho, Higashi-ku Nagoya, Aichi-ken(JP)**

Inventor: **Mitani, Takahiko, c/o Sanwa Kagaku Kenkyusho Co.**
**Limited, 35, Higashi-sotobori-cho, Higashi-ku Nagoya, Aichi-ken(JP)**
Inventor: **Takahashi, Haruo, c/o Sanwa Kagaku Kenkyusho Co.**
**Limited, 35, Higashi-sotobori-cho, Higashi-ku Nagoya, Aichi-ken(JP)**
Inventor: **Sawai, Kiichi, c/o Sanwa Kagaku Kenkyusho Co.**
**Limited, 35, Higashi-sotobori-cho, Higashi-ku Nagoya, Aichi-ken(JP)**

(74) Representative: **Wächtershäuser, Günter, Dr.**
**Tal 29**
**W-8000 München 2(DE)**

(54) Calcitonin analogues and use thereof.

(57) There are disclosed polypeptides with biological activities similar to calcitonin and its raw material. The polypeptides are shown by general formula (I) of

$$\text{H-Cys- } A_1\text{-Asn-Leu-Ser-Thr-Cys- } A_2\text{-Leu-Gly- } A_3\text{- } A_4\text{- } A_5\text{-}$$
$$\text{Gln- } A_6\text{- } A_7\text{- } A_8\text{-Lys- } A_9\text{-}A_{10}\text{-Thr-}A_{11}\text{-Pro-}A_{12}\text{-Thr-}A_{13}\text{-}$$
$$A_{14}\text{-Gly-}A_{15}\text{-Gly-}A_{16}\text{-Pro- } Y$$

wherein $A_1$ is Ser, Gly or Ala; $A_2$ is Val or Met; $A_3$ is Thr or Lys; $A_4$ is Tyr or Leu; $A_5$ is Thr or Ser; $A_6$ is Asp or Glu; $A_7$ is Phe or Leu; $A_8$ is Asn or His; $A_9$ is Tyr, Phe or Leu; $A_{10}$ is His or Gln; $A_{11}$ is Tyr or Phe; $A_{12}$ is Gln or Arg; $A_{13}$ is Ala, Ser, Asn or Asp; $A_{14}$ is Ile, Thr or Val; $A_{15}$ is Val, Ser or Ala; $A_{16}$ is Ala, Thr or Val; and Y is amidized homoserine, homoserine-lactone residue reacted with a primary alkyl amine containing 1 - 20 carbon atoms or an optional polypeptide chain and containing an amidized homoserine at C-terminal.
The polypeptide can be used as an effective ingredient for medicines to cure calcium metabolic diseases.

EP 0 464 549 A1

Rank Xerox (UK) Business Services

The present invention relates to a calcitonin analogue, namely a novel polypeptide with biological activities as in "calcitonin", use thereof and a raw material for preparing the same.

The calcitonin analogue according to the invention can be used as an effective ingredient for medicines, especially for curing calcium metabolic diseases, for instance a hypercalcemia and bone betzettis diseases as well as for reducing of pain and suppressing a decrease of bone in osteoporosis.

In the year of 1961, D. H. Copp et al had reported that in blood of human cervical vein, there is a substance with an action to reduce calcium concentration in blood and they named the substance as "calcitonin". In 1963, P. F. Hirsch et al have found that a substance having similar activities to the calcitonin presents in a thyroid gland of rats. Since a structure of porcine calcitonin was determined in 1968 by J. T. Potts et al, 10 kinds of substances with activities similar to those of the calcitonin have been extracted and purified from blood or-tissues of other 7 kinds of animals, namely human being, cattle, sheep, rat, salmon, rabbit and domestic fowl, and an amino acid structure on those substances has been determined in said order.

Amino acid sequences for human, porcine, salmon and eel calcitonins are given below [In the sequences, symbol (*) shows that it is same with that in the sequence for human calcitonin].

```
                        1                5                10
      Human      : H-Cys-Gly-Asn-Leu-Ser-Thr-Cys-Met-Leu-Gly-Thr-
      Porcine :      *  Ser  *   *   *   *   *  Val  *  Ser Ala
      Salmon  :      *  Ser  *   *   *   *   *  Val  *   *  Lys
      Eel     :      *  Ser  *   *   *   *   *  Val  *   *  Lys
```

```
                               15                20
      Human      :   Tyr-Thr-Gln-Asp-Phe-Asn-Lys-Phe-His-Thr-Phe-
      Porcine :       *  Trp Arg Asn Leu  *  Asn  *   *  Arg  *
      Salmon  :      Leu Ser  *  Glu Leu His  *  Leu Gln  *  Tyr
      Eel     :      Leu Ser  *  Glu Leu His  *  Leu Gln  *  Tyr
```

```
                             25                30
      Human      :   Pro-Gln-Thr-Ala-Ile-Gly-Val-Gly-Ala-Pro-NH₂
      Porcine :      Ser Gly Met Gly Phe  *  Pro Glu Thr  *   *
      Salmon  :       *  Arg  *  Asn Thr  *  Ser  *  Thr  *   *
      Eel     :       *  Arg  *  Asp Val  *  Ala  *  Thr  *   *
```

Activities of the calcitonins are given in following Table.

2

| Calcitonins | Organ | MRCU/mg |
|---|---|---|
| Human | thyroid grand | 70 |
| Porcine | ditto | 120 |
| Salmon | back gland of branchia | 2700 |
| Eel | ditto | 3500 - 5000 |

In the Table,

MRCU : Medical Research Council Unit.

The calcitonin is one of polypeptide hormones, consists of 32 amino acids, and has structural characteristics in that cysteines at N-terminal and in 7th position make the polypeptide into a ring structure with a disulfide bond therebetween and that there is an amide structure at C-terminal. As biological activities of the calcitonin, a reduction of calcium concentration in blood, acceleration of bone formation, acceleration of phosphate elimination into urine, suppression of gastric juice secretion and the like have been known, but most important activity is an adjustment of calcium metabolism in living body. Among the structural characteristics, the amide structure at C-terminal of the calcitonin is indispensable for developing its activity, since if its portion shall be hydrolyzed, the calcitonin loses its activity as hormone.

For obtaining a polypeptide having an amide structure at C-terminal by expressing the polypeptide with use of a microorganism such as Escherichia coli with use of conventional techniques, in general, there is required a special technique of using C-terminal amidation enzyme, when the expressed polypeptide shall be isolated and purified. However, such enzyme is expensive and the enzymatic process makes yield of the objective substance low, so that such conventional process can not be said as one suitable for industrial scale production of the polypeptide.

Although there is no relation to the calcitonin in question, the inventors have found that on motilin analogues accelerating a peristalsis of intestines, those with homoserine or homoserine-lactone at C-terminal show biological activities in same level with or more higher than the native motilin, and they have proposed a process for preparing such motilin analogues with a reasonable cost and in a large amount [Jap. Pat. Appln. No. Sho 64 (1989) - 286, corresponding to a part of USSN 07/459236 and EP-03 78 078-(A1)].

Hitherto, it has been considered as quite difficult to provide the calcitonin at a reasonable price and in a large amount, since according to the prior arts, there is no way for obtaining the calcitonin other than a synthetic process therefor or an extraction thereof from an animal blood or tissue, and the former requires troublesome operations due to that the calcitonin is polypeptide consisting of 32 amino acids, and takes a relatively long period of time in its chemical synthesis and for purifying the same, and the latter is restricted on availability of the raw material and requires troublesome purifying operations.

Further, the technique utilizing so-called "Biotechnology" can not be said as --convenient method--, since it requires a special technique for the amidation at C-terminal, as referred to hereinbefore.

A main object of the invention is to provide a calcitonin analogue with biological activities in same level with or more higher than native calcitonin, by chemically synthesizing a polypeptide with a calcitonin-like structure, cleaving the resulting polypeptide with cyanogen bromide to prepare another polypeptide with homoserine (inclusive of homoserine-lactone) at C-terminal and calcitonin-like structure (synthesis of raw material for preparing the calcitonin analogue with biological activities), and then carrying out a simple chemical reaction thereon.

An additional but important object of the invention is to provide a pharmaceutical composition for curing calcium metabolic diseases, which contains as an effective ingredient the calcitonin analogue.

Another object of the invention is to provide a polypeptide which is useful as a raw material for preparing the pharmacologically active calcitonin analogue in good efficiency and at a reasonable cost.

The inventors have energetically studied and investigated on polypeptides obtained through a fermentation method or chemical synthesis and with a calcitonin-like structure. As a result, they have found that a polypeptide obtained through the cleaving step using cyanogen bromide has homoserine residue (inclusive of homoserine-lactone residue) at C-terminal, that an amide or fatty amine can easily be bond to the residue in accordance with a conventional synthetic method, and that the resulting calcitonin analogue shows biological activities in same level with or more higher than the native calcitonin, to establish the invention.

Therefore, according to the invention, problems in the prior arts can be dissolved by a calcitonin analogue shown by the general formula (I) of

$$\text{H-Cys- } A_1\text{-Asn-Leu-Ser-Thr-Cys- } A_2\text{-Leu-Gly- } A_3\text{- } A_4\text{- } A_5\text{-}$$
$$\text{Gln- } A_6\text{- } A_7\text{- } A_8\text{-Lys- } A_9\text{-}A_{10}\text{-Thr-}A_{11}\text{-Pro-}A_{12}\text{-Thr-}A_{13}\text{-}$$
$$A_{14}\text{-Gly-}A_{15}\text{-Gly-}A_{16}\text{-Pro- } Y$$

wherein $A_1$ is Ser, Gly or Ala; $A_2$ is Val or Met; $A_3$ is Thr or Lys; $A_4$ is Tyr or Leu; $A_5$ is Thr or Ser; $A_6$ is Asp or Glu; $A_7$ is Phe or Leu; $A_8$ is Asn or His; $A_9$ is Tyr, Phe or Leu; $A_{10}$ is His or Gln; $A_{11}$ is Tyr or Phe; $A_{12}$ is Gln or Arg; $A_{13}$ is Ala, Ser, Asn or Asp; $A_{14}$ is Ile, Thr or Val; $A_{15}$ is Val, Ser or Ala; $A_{16}$ is Ala, Thr or Val; and Y is amidized homoserine, homoserine-lactone reacted with a primary alkyl amine containing 1 - 20 carbon atoms or an optional polypeptide chain and containing an amidized homoserine at C-terminal,
to attain the main object as referred to.

The additional object as referred to can be attained by a pharmaceutical composition for curing calcium metabolic diseases, which contains as an effective ingredient the calcitonin analogue in an effective amount.

A polypeptide according to the invention for preparing the calcitonin analogue shown by Formula (I) is shown by the general formula (II) of

$$\text{H-Cys- } A_1\text{-Asn-Leu-Ser-Thr-Cys- } A_2\text{-Leu-Gly- } A_3\text{- } A_4\text{- } A_5\text{-}$$
$$\text{Gln- } A_6\text{- } A_7\text{- } A_8\text{-Lys- } A_9\text{-}A_{10}\text{-Thr-}A_{11}\text{-Pro-}A_{12}\text{-Thr-}A_{13}\text{-}$$
$$A_{14}\text{-Gly-}A_{15}\text{-Gly-}A_{16}\text{-Pro- } Y'$$

wherein $A_1$, $A_2$, $A_3$, $A_4$, $A_5$, $A_6$, $A_7$, $A_8$, $A_9$, $A_{10}$, $A_{11}$, $A_{12}$, $A_{13}$, $A_{14}$, $A_{16}$ and $A_{16}$ have the meanings as referred to; and Y' is homoserine (inclusive of homoserine-lactone and indicated with a symbol of "Hse").

The invention will now be further explained in more detail with reference to Manufacturing Example, Pharmacological Test Example as well as Medicine Preparation Example.

In the followings, the explanation will be given on salmon calcitonin analogue, but please note that other and various calcitonin analogues having valine residue at 8th position ($A_2$ position of Formulae I) can be prepared in a similar manner by using a fermentation method, and that a calcitonin analogue having methionine residue at 8th position may also be prepared by protecting the methionine residue with use of a chemical synthetic method to prevent a cleaving at that position, then treating with cyanogen bromide to form homoserine or homoserine-lactone residue at C-terminal, and thereafter removing the protecting group for the methionine residue.

Example 1

(Synthesis of calcitonin analogues)

In the first place, a polypeptide encoding the following amino acid sequence was synthesized with use of a peptide synthesizer (Type 430A marketed by Applied Biosystems Co.).

$$\text{H-Cys-Ser-Asn-Leu-Ser-Thr-Cys-Val-Leu-Gly-Lys-Leu-Ser-Gln-}$$
$$\text{Glu-Leu-His-Lys-Leu-Gln-Thr-Tyr-Pro-Arg-Thr-Asn-Thr-Gly-}$$
$$\text{Ser-Gly-Thr-Pro-Met-Ala-Ser}$$

A purification of the synthesized polypeptide was carried out by subjecting to HPLC with use of a micropondasphere C-18 column (19mm x 15cm) marketed by Waters Co.

The resulting purified sample (10mg) was taken and dissolved in 70% formic acid solution (30ml). After added 50mg of cyanogen bromide, the solution was kept for 24 hours at 37°C to cause a reaction. Then, distilled water (200ml) was added to the reaction mixture and lyophilized to remove the formic acid and cyanogen bromide. The resulting material was subjected again to HPLC under the following conditions, with use of micropondasphere C-18 column (19mm x 15cm) marketed by Waters Co.

Elute :     Linear gradient of 20% to 70% acetonitrile in 0.1% trifluoroacetic acid (30 minutes),
Flow rate :     7.0ml/min.

Fractions in a main peak part on the HPLC were recovered and lyophilized. Through said proceedings, the synthesized polypeptide encoding said amino acid sequence has been cleaved at methionine residue part at 33rd position and it is modified into homoserine or homoserine-lactone residue at C-terminal.

If the polypeptide is further treated with an acid (for instance, in 0.1N HCl at 30°C for 3 hours) and then lyophilized, the homoserine residue at C-terminal can be converted into the homoserine-lactone residue in the level of 70% or more.

The polypeptide with the homoserine-lactone residue at C-terminal was collected through HPLC and lyophilized. The resulting dried powder was treated 10% ammonia in dimethylformamide solution at room temperature for 24 hours to prepare a desired calcitonin analogue with homeserine-amide residue at C-terminal.

A part of the resulting calcitonin analogue was taken and checked with use of a peptide sequencer marketed by Applied Biosystems Co. to confirm that the initially synthesized polypeptide is cleaved at correct position of methionine residue at 33rd position and the homoserine-lactone residue at C-terminal has been amidized.

Another type calcitonin analogue with an alkyl amine residue at C-terminal was prepared by reacting the polypeptide with homoserine-lactone residue at C-terminal with $CH_3(CH_2)_9NH_2$, $CH_3(CH_2)_{19}NH_2$ or the like primary fatty amine, in dimethylsulfoxide solution.

Still other type calcitonin analogue with a homoserineamide residue at C-terminal was prepared by cleaving a calcitonin containing protein expressed with Escherichia coli with cyanogen bromide and then reacting the resulting polypeptide with ammonia or a primary alkyl amine.

Biological Activity Test Example

A solution to be tested was diluted with 0.1% sodium acetate solution (pH 4.0, containing 0.1% cattle serum albumin) and injected to a male rat (body weight : about 100g) by 0.1ml/animal from its tail vein. After 1 hour from the injection, a blood letting was carried out from the heart to measure a calcium concentration in its serum through a spectroscopic method with use of calcium measuring kit marketed by Wako Junyaku Kabushiki Kaisha of Japan. By utilizing the method, an amount of the calcitonin analogues to be required for reducing or lowering a calcium concentration in serum by 10% was measured. The amount [to be defined as 10% MRCU (Medical Research Council Unit)] are shown below.

A) A group (named "h-Ca Group") of the compounds of Formula I, wherein $A_1$ is Gly, $A_2$ is Val, $A_3$ is Thr, $A_4$ is Tyr, $A_5$ is Thr, $A_6$ is Asp, $A_7$ is Phe, $A_8$ is Asn, $A_9$ is Phe, $A_{10}$ is His, $A_{11}$ is Phe, $A_{12}$ is Gln, $A_{13}$ is Ala, $A_{14}$ is Ile, $A_{11}$ is Val, and $A_{16}$ is Ala, and in which
    a) Y is Hse-$NH_2$ : 100 MRCU/mg;
    b) Y is Hse-$NH(CH_2)_9CH_3$ : 110 MRCU/mg; and
    c) Y is Hse-$NH(CH_2)_{19}CH_3$ : 90 MRCU/mg.

B) Another group (named "s-Ca Group") of the compounds of Formula I, wherein $A_1$ is Ser, $A_2$ is Val, $A_3$ is Lys, $A_4$ is Leu, $A_5$ is Ser, $A_6$ is Glu, $A_7$ is Leu, $A_8$ is His, $A_9$ is Leu, $A_{10}$ is Gln, $A_{11}$ is Tyr, $A_{12}$ is Arg, $A_{13}$ is Asn, $A_{14}$ is Thr, $A_{15}$ is Ser, and $A_{16}$ is Thr, and in which
    a) Y is Hse-$NH_2$ :
    3000 MRCU/mg;
    b) Y is Hse-$NH(CH_2)_9CH_3$ :
    2900 MRCU/mg; and
    c) Y is Hse-$NH(CH_2)_{19}CH_3$ :
    2800 MRCU/mg.

C) Still other group (named "e-Ca Group") of the compounds of Formula I, wherein $A_1$ is Ser, $A_2$ is Val, $A_3$ is Lys, $A_4$ is Leu, $A_5$ is Ser, $A_6$ is Asp, $A_7$ is Leu, $A_8$ is His, $A_9$ is Leu, $A_{10}$ is Gln, $A_{11}$ is Phe, $A_{12}$ is Arg, $A_{13}$ is Asp, $A_{14}$ is Val, $A_{16}$ is Ala, and $A_{16}$ is Val, and in which
    a) Y is Hse-$NH_2$ :

6000 MRCU/mg;
b) Y is Hse-NH(CH$_2$)$_9$CH$_3$ :
6200 MRCU/mg; and
c) Y is Hse-NH(CH$_2$)$_{19}$CH$_3$ :
5900 MRCU/mg.

<u>Medicine</u> <u>Preparation</u> <u>Example</u>

A solution of the calcitonin analogue according to the invention (the polypeptide A-a described in the Biological Activity Test Example) in refined water was aseptically charged into vials, so that each vial contains the polypeptide by 1mg. After lyophilized, the vial was sealed to obtain a dry powdery medicine. The powdery medicine is dissolved in saline or the like for injection purpose, when it shall be used.

For stabilizing the calcitonin analogue, a human serum albumin or the like can be used.

## Claims

1.  A calcitonin analogue shown by the general formula (I) of

$$\text{H-Cys-} A_1\text{-Asn-Leu-Ser-Thr-Cys-} A_2\text{-Leu-Gly-} A_3\text{-} A_4\text{-} A_5\text{-}$$
$$\text{Gln-} A_6\text{-} A_7\text{-} A_8\text{-Lys-} A_9\text{-}A_{10}\text{-Thr-}A_{11}\text{-Pro-}A_{12}\text{-Thr-}A_{13}\text{-}$$
$$A_{14}\text{-Gly-}A_{15}\text{-Gly-}A_{16}\text{-Pro-} Y$$

wherein $A_1$ is Ser, Gly or Ala; $A_2$ is Val or Met; $A_3$ is Thr or Lys; $A_4$ is Tyr or Leu; $A_5$ is Thr or Ser; $A_6$ is Asp or Glu; $A_7$ is Phe or Leu; $A_8$ is Asn or His; $A_9$ is Tyr, Phe or Leu; $A_{10}$ is His or Gln; $A_{11}$ is Tyr or Phe; $A_{12}$ is Gln or Arg; $A_{13}$ is Ala, Ser, Asn or Asp; $A_{14}$ is Ile, Thr or Val; $A_{15}$ is Val, Ser or Ala; $A_{16}$ is Ala, Thr or Val; and Y is amidized homoserine, homoserine-lactone reacted with a primary alkyl amine containing 1 - 20 carbon atoms or an optional polypeptide chain and containing an amidized homoserine at C-terminal.

2.  A pharmaceutical composition for curing calcium metabolic diseases, which contains as an effective ingredient a calcitonin analogue shown by the general formula (I) of

$$\text{H-Cys-} A_1\text{-Asn-Leu-Ser-Thr-Cys-} A_2\text{-Leu-Gly-} A_3\text{-} A_4\text{-} A_5\text{-}$$
$$\text{Gln-} A_6\text{-} A_7\text{-} A_8\text{-Lys-} A_9\text{-}A_{10}\text{-Thr-}A_{11}\text{-Pro-}A_{12}\text{-Thr-}A_{13}\text{-}$$
$$A_{14}\text{-Gly-}A_{15}\text{-Gly-}A_{16}\text{-Pro-} Y$$

wherein $A_1$ is Ser, Gly or Ala; $A_2$ is Val or Met; $A_3$ is Thr or Lys; $A_4$ is Tyr or Leu; $A_5$ is Thr or Ser; $A_6$ is Asp or Glu; $A_7$ is Phe or Leu; $A_8$ is Asn or His; $A_9$ is Tyr, Phe or Leu; $A_{10}$ is His or Gln; $A_{11}$ is Tyr or Phe; $A_{12}$ is Gln or Arg; $A_{13}$ is Ala, Ser, Asn or Asp; $A_{14}$ is Ile, Thr or Val; $A_{15}$ is Val, Ser or Ala; $A_{16}$ is Ala, Thr or Val; and Y is amidized homoserine, homoserine-lactone reacted with a primary alkyl amine containing 1 - 20 carbon atoms or an optional polypeptide chain and containing an amidized homoserine at C-terminal, in an effective amount.

3.  A polypeptide shown by the general formula (II) of

$$\text{H-Cys-} A_1\text{-Asn-Leu-Ser-Thr-Cys-} A_2\text{-Leu-Gly-} A_3\text{-} A_4\text{-} A_5\text{-}$$
$$\text{Gln-} A_6\text{-} A_7\text{-} A_8\text{-Lys-} A_9\text{-}A_{10}\text{-Thr-}A_{11}\text{-Pro-}A_{12}\text{-Thr-}A_{13}\text{-}$$
$$A_{14}\text{-Gly-}A_{15}\text{-Gly-}A_{16}\text{-Pro-} Y'$$

wherein $A_1$ is Ser, Gly or Ala; $A_2$ is Val or Met; $A_3$ is Thr or Lys; $A_4$ is Tyr or Leu; $A_5$ is Thr or Ser; $A_6$ is Asp or Glu; $A_7$ is Phe or Leu; $A_8$ is Asn or His; $A_9$ is Tyr, Phe or Leu; $A_{10}$ is His or Gln; $A_{11}$ is Tyr or

Phe; $A_{12}$ is Gln or Arg; $A_{13}$ is Ala, Ser, Asn or Asp; $A_{14}$ is Ile, Thr or Val; $A_{15}$ is Val, Ser or Ala; $A_{16}$ is Ala, Thr or Val; and Y is homoserine or homoserine-lactone.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | BIO/TECHNOLOGY, vol. 6, no. 2, February 1988, pages 190-192; T. KEMPE et al.: "[Homoserine31]-salmon cal-citonin I: Fully active analogue of calcitonin synthesized by recombinant DNA techniques" * Whole document * | 1-3 | C 07 K 7/10 A 61 K 37/02 |
| | — — — | | |
| A | US-A-4 658 014   (KEMPE) * Claim 1 * | 1-3 | |
| | — — — — — | | |

TECHNICAL FIELDS
SEARCHED (Int. Cl.5)

C 07 K
A 61 K

The present search report has been drawn up for all claims

| Place of search | Date of completion of search | Examiner |
|---|---|---|
| The Hague | 09 October 91 | MASTURZO P. |

CATEGORY OF CITED DOCUMENTS

X : particularly relevant if taken alone
Y : particularly relevant if combined with another
   document of the same catagory
A : technological background
O : non-written disclosure
P : intermediate document
T : theory or principle underlying the invention

E : earlier patent document, but published on, or after
   the filing date
D : document cited in the application
L : document cited for other reasons

& : member of the same patent family, corresponding
   document